# EUROPÄISCHE PATENTANMELDUNG

(11) **EP 0 789 067 A1**
(43) Veröffentlichungstag der Anmeldung: **13.08.1997**
(21) Anmeldenummer: 97101762.9
(22) Anmeldetag: 05.02.1997
(51) Int. Cl.: C09K 19/34, C09K 19/30, C09K 19/42, C07D 319/06, C07D 239/26, C07D 239/28, C07D 239/34, C07D 213/26, C07D 213/65, C07C 25/02, C07C 43/225, C07C 255/50, C07C 255/54, C07C 255/55, C07C 69/78, C07C 69/75

(54) **Cyclopentyl-Derivate als Flüssigkristalle**

(30) Priorität: 12.02.1996 CH 353/96
(71) Anmelder: Rolic AG, 4058 Basel (CH)
(72) Erfinder: Buchecker, Richard, 8008 Zurich (CH); Villiger, Alois, 4056 Basel (CH)
(74) Vertreter: Kjellsaa-Berger, Hanny, Dr.

(57) **Zusammenfassung**

Die vorliegende Erfindung betrifft flüssigkristalline Verbindungen der allgemeinen Formel 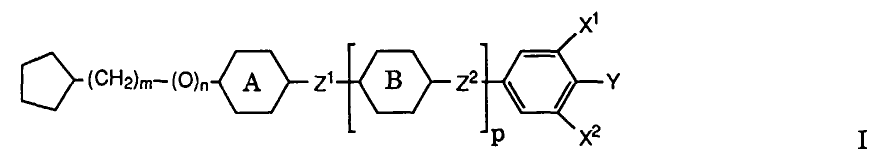 worin die
- Ringe A und B: unabhängig voneinander trans-1,4-Cyclohexylen oder gegebenenfalls einfach oder mehrfach mit Fluor substituiertes 1,4-Phenylen oder einer der Ringe A und B auch trans-1,3-Dioxan-5,2-diyl, Pyridin-5,2-diyl oder Pyrimidin-5,2-diyl;
- Z¹ und Z²: eine Einfachbindung oder eine der Gruppen auch -(CH₂)₂-, -CH₂O-, -COO- oder -C≡C-;
- X¹ und X²: Wasserstoff oder Fluor;
- Y: Fluor, Chlor, Cyano, -OCHF₂, -CHF₂, -OCF₃, oder -CF₃;
- m: eine ganze Zahl von 0 bis 7;
- n: 0 oder, falls A ein aromatischer Ring ist, auch 1; und
- p: 0 oder 1 bedeuten;
flüssigkristalline Mischungen enthaltend solche Verbindungen sowie die Verwendung solcher Verbindungen und Mischungen in optischen Vorrichtungen.

## Beschreibung

Die vorliegende Erfindung betrifft neuartige flüssigkristalline Verbindungen mit einer endständigen Cyclopentyl-Gruppe, flüssigkristalline Mischungen enthaltend solche Verbindungen sowie die Verwendung solcher Verbindungen und Mischungen in optischen Vorrichtungen.

Flüssige Kristalle werden vor allem als Dielektrika in Anzeigevorrichtungen verwendet, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gas/Wirt-Zellen, TN-Zellen mit verdrillt nematischer ("twisted nematic") Struktur, STN-Zellen ("super twisted nematic"), SBE-Zellen ("super birefringence effect") und OMI-Zellen ("optical mode interference"). Für Displays mit hohem Informationsgehalt sind in letzter Zeit neben den passiv angesteuerten, multiplexierten Zellen, besonders die aktiv angesteuerten, z.B. TFT-Zellen ("thin film transistor"), wichtig geworden. Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische, photochemische und thermische Stabilität und eine gute Stabilität gegenüber elektrischen Feldern besitzen. Ferner sollten sie über einen möglichst breiten Bereich eine geeignete Mesophase aufweisen (beispielsweise für die oben genannten Zellen eine nematische bzw. eine cholesterische Phase), sie sollten aber trotzdem eine ausreichend niedere Viskosität besitzen, um in den Zellen kurze Ansprechzeiten, tiefe Schwellenspannungen und einen hohen Kontrast zu ermöglichen. Weitere Eigenschaften, wie die elektrische Leitfähigkeit, die dielektrische Anisotropie und die optische Anisotropie, müssen je nach Anwendungsgebiet und Zellentyp unterschiedlichen Anforderungen genügen. Beispielsweise sollten Materialien für die Zellen mit verdrillt nematischer Struktur eine möglichst hohe positive dielektrische Anisotropie und gleichzeitig eine möglichst geringe Leitfähigkeit aufweisen. Die letztere Eigenschaft ist vor allem für aktiv adressierte Flüssigkristallanzeigen mit hoher Informationsdichte, z.B. TFT-Zellen, von besonderer Wichtigkeit. Leider führen aber Komponenten mit höherer dielektrischer Anisotropie infolge ihres besseren Lösungsvermögens für ionische Verunreinigungen meist zu einer erhöhten Leitfähigkeit in Mischungen. Es werden daher Komponenten gesucht, die sich durch eine möglichst hohe dielektrische Anisotropie bei gleichzeitig möglichst geringer Leitfähigkeit auszeichnen.

Ueberraschenderweise wurde gefunden, dass flüssigkristalline Verbindungen mit einer Cyclopentyl-Gruppe am Ende einer Seitenkette oder aber anstelle einer Seitenkette sich durch einen erhöhten Klärpunkt oder auch eine verbesserte Löslichkeit auszeichnen. Zudem werden die positiven Eigenschaften wie chemische Stabilität, Viskosität, dielektrische und optische Anisotropie usw. durch die Cyclopentyl-Gruppe kaum beeinträchtigt.

Die vorliegende Anmeldung betrifft somit Verbindungen der allgemeinen Formel 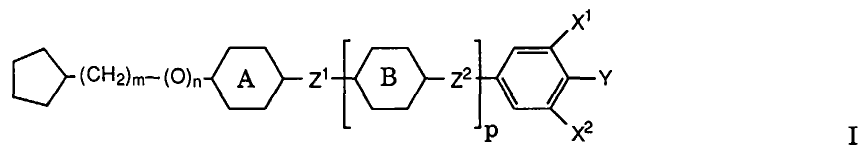 worin die
- Ringe A und B: unabhängig voneinander trans-1,4-Cyclohexylen oder gegebenenfalls einfach oder mehrfach mit Fluor substituiertes 1,4-Phenylen, oder einer der Ringe A und B auch trans-1,3-Dioxan-5,2-diyl, Pyridin-5,2-diyl oder Pyrimidin-5,2-diyl;
- Z¹ und Z²: eine Einfachbindung oder eine der Gruppen Z¹ und Z² auch -(CH₂)₂-, -CH₂O-, -COO- oder -C≡C-;
- X¹ und X²: unabhängig voneinander Wasserstoff oder Fluor;
- Y: Fluor, Chlor, Cyano, -OCHF₂, -CHF₂, -OCF₃ oder -CF₃;
- m: eine ganze Zahl von 0 bis 7;
- n: 0 oder, falls A ein aromatischer Ring ist, auch 1; und
- p: 0 oder 1 bedeuten.

Die erfindungsgemässen Verbindungen, worin m + n = 0 oder eine gerade Zahl ergeben, zeichnen sich durch einen vergleichsweisen hohen Klärpunkt aus, ohne dass dabei die Löslichkeit dieser Verbindungen in flüssigkristallinen Mischungen herabgesetzt wird, besonders bevorzugt sind Verbindungen der Formel I, worin m = 0, 2 oder 4 und n = 0 ist. Bei Verbindungen der Formel I, worin m + n = eine ungerade Zahl ergeben, ist insbesondere die Löslichkeit in flüssigkristallinen Mischungen im Vergleich zu konventionellen flüssigkristallinen Verbindungen stark verbessert.

Besonders bevorzugt werden Verbindungen der Formel I, worin Ring A trans-1,4-Cyclohexylen, gegebenenfalls einfach oder mehrfach mit Fluor substituiertes 1,4-Phenylen, trans-1,3-Dioxan-5,2-diyl oder Pyrimidin-5,2-diyl; und Ring B trans-1,4-Cyclohexylen oder gegebenenfalls einfach oder mehrfach mit Fluor substituiertes 1,4-Phenylen bedeuten. Dabei bedeutet der Ausdruck "gegebenenfalls einfach oder mehrfach mit Fluor substituiertes 1,4-Phenylen", 1,4-Phenylen, 2- bzw. 3-Fluor-1,4-Phenylen oder 2,6- bzw. 3,5-Difluor-1,4-phenylen.

Weiter werden Verbindungen der Formel I bevorzugt, worin p = 1 ist, es sind dies insbesondere Verbindungen der allgemeinen Formeln 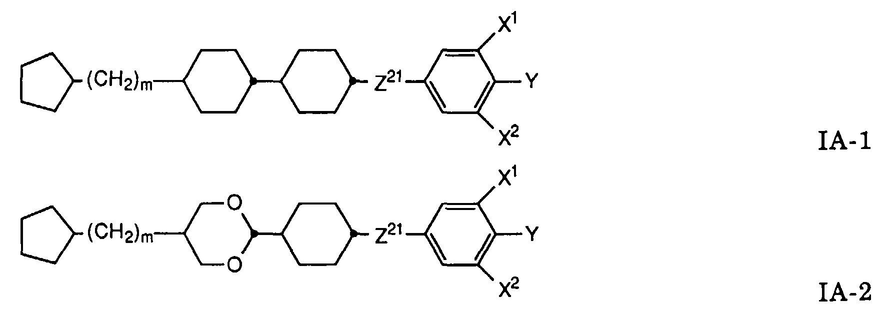 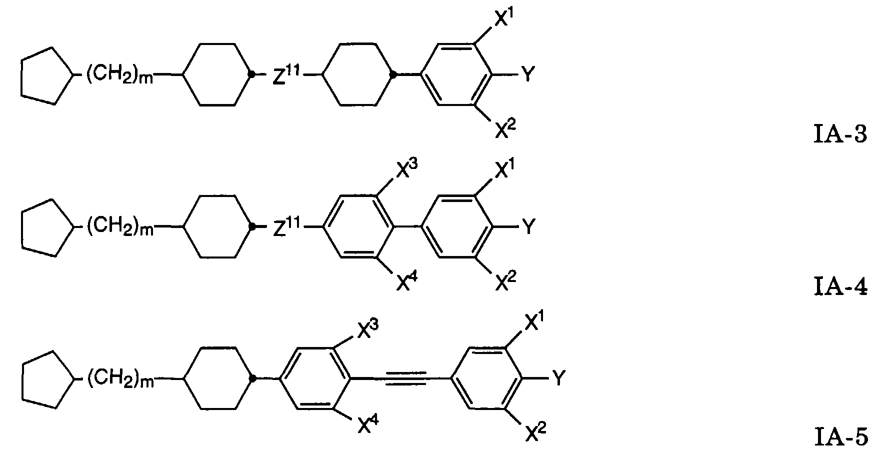 worin
- Z¹¹ und Z²¹: eine Einfachbindung oder -(CH₂)₂-;
- X³ und X⁴: unabhängig voneinander Wasserstoff oder Fluor bedeuten; und
- m, Y, X¹ und X²: die obengenannten Bedeutungen haben.

Besonders bevorzugt werden die Verbindungen der Formeln IA-1 bis IA-5, worin m eine ganze Zahl von 0 bis 4, insbesondere jedoch 0, 2 oder 4 bedeutet.

Die Verbindungen der Formeln IA-1 bis IA-5 eignen sich für passiv adressierte Anzeigevorrichtungen, insbesondere, wenn Y Cyano bedeutet. Verbindungen der Formeln IA-1 bis IA-4, worin Y Fluor, -CF₃, -OCHF₂ oder -OCF₃ bedeuten, zeichnen sich hingegen durch hervorragende Eigenschaften aus, welche sie hauptsächlich für die Anwendung in aktiv adressierten Anzeigevorrichtungen qualifizieren. In ganz besonders bevorzugten Verbindungen der Formeln IA-1 bis IA-5 bedeutet Y Fluor.

Ein weiterer Aspekt der Erfindung betrifft Verbindungen der allgemeinen Formel I, worin p = 0 ist; es sind dies insbesondere die Verbindungen der allgemeinen Formeln 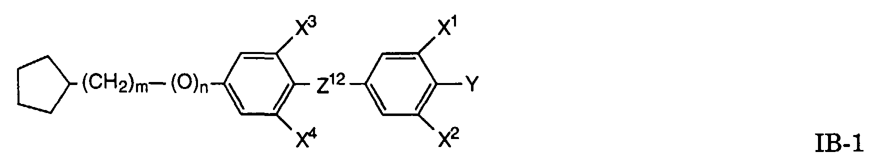 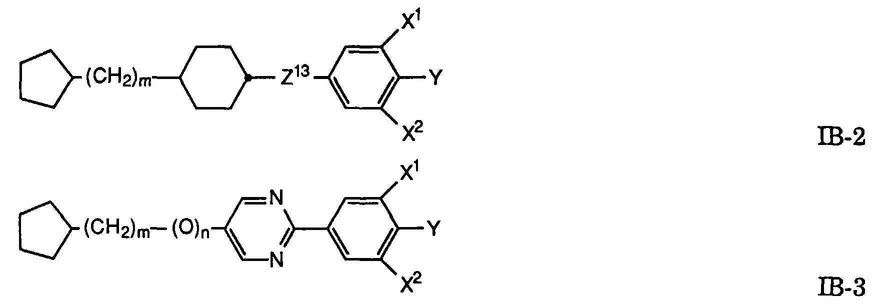 worin
- m: eine ganze Zahl von 0 bis 7 ist;
- n: 0 oder 1 ist;
- X³ und X⁴: unabhängig voneinander Wasserstoff oder Fluor;
- Z¹²: eine Einfachbindung, -COO- oder -C≡C-;
- Z¹³: eine Einfachbindung, -(CH₂)₂-, -CH₂O- oder -COO- bedeuten; und
- Y, X¹ und X²: die obengenannten Bedeutungen haben.

Ganz besonders bevorzugt sind die Verbindungen der Formeln IB-1, worin Z¹² -COO-, Y Cyano und X¹ Fluor bedeuten, insbesondere die Verbindungen der allgemeinen Formel 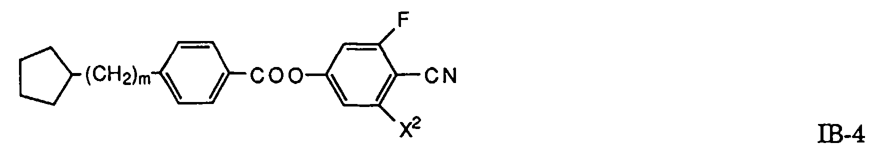 worin m und X² die obengenannten Bedeutungen haben.

Die Verbindungen der Formeln IB-1 bis IB-3 und insbesondere Verbindungen der Formel IB-4 zeichnen sich durch eine hohe dielektrische Anisotropie aus und eignen sich daher sehr gut für die Anwendung in passiv adressierten Anzeigevorrichtungen. Die Verbindungen IB-2, worin Z¹³ eine Einfachbindung oder -(CH₂)₂ und Y Fluor, -CF₃, -OCHF₂ oder -OCF₃ bedeuten, eignen sich insbesondere für aktiv adressierte Anzeigevorrichtungen.

Die erfindungsgemässen Verbindungen der Formel I können in an sich bekannter Weise hergestellt werden. So werden Verbindungen, worin A = trans-1,4-Cyclohexylen und m = 0 oder 1 bedeuten, vorzugsweise über eine Wittig-Reaktion eines 4-substituierten Cyclohexanons mit einem Wittigsalz eines Halo- bzw. Halomethyl-cyclopentans mit Triphenylphosphin unter Zusatz eines Aequivalents einer geeigneten Base, wie z.B. Kalium-tert. butylat, in einem inerten Lösungsmittel, vorzugsweise zwischen -20°C und Raumtemperatur, und anschliessender katalytischer Hydrierung vorzugsweise mit Platin/Kohle in Ethanol, Ethanol/Toluol-Gemischen und dergleichen, erhalten.

Verbindungen, worin n = 0 ist, m eine ganze Zahl von 2 bis 6 bedeutet und Ring A trans-1,4-Cyclohexylen, gegebenenfalls substituiertes 1,4-Phenylen, Pyrimidin-5,2-diyl oder Pyridin-5,2-diyl bedeutet, werden vorzugsweise durch eine Wittig-Reaktion einer Formylverbindung mit den entsprechenden Cyclopentylalkyl-Wittigsalzen und anschliessender katalytischen Hydrierung mit Palladium/Kohle z.B. in Toluol hergestellt.

Verbindungen, worin Ring A gegebenenfalls substituiertes 1,4-Phenylen, p = 0 und m = 1 ist, werden vorzugsweise durch eine Friedel-Crafts-Acylierung des entsprechenden Phenylderivates mit einem Cyclopentanoylchlorid und Aluminiumchlorid, z.B. in Dichlormethan, mit Reduktion des Cyclopentanoylbenzols, z.B. nach Huang-Minlon, hergestellt.

Verbindungen, worin Ring A gegebenenfalls substituiertes 1,4-Phenylen und p und m = 0 bedeuten, werden vorzugsweise ausgehend aus dem durch Friedel-Crafts-Alkylierung von Benzol mit Bromcyclopentan in Anwesenheit von Aluminumchlorid erhältlichem Cyclopentylbenzol hergestellt.

Verbindungen, worin Ring A trans-1,3-Dioxan-5,2-ylen bedeutet, werden vorzugsweise durch Umsetzen eines Natriummalonats (Herstellung in situ aus unsubstituiertem Malonester mit Natriumalkylat, wie Natriummethylat oder Natriumethylat) mit einem entsprechenden Brom- oder (ω-Bromalkyl)-cyclopentan, Reduktion des erhaltenen alkylierten Malonesters mit Lithiumaluminiumhydrid zum 2-alkylierten 1,3-Propandiol, säurekatalysierte Umsetzung des Diols mit einem Aldehyd der Formel 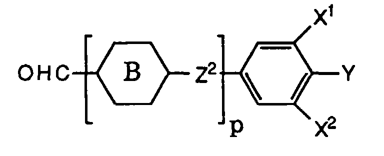 worin die Symbole die obengenannten Bedeutungen haben,
in einem inerten Lösungsmittel, wie beispielsweise Benzol oder Toluol, vorzugsweise bei Siedetemperatur hergestellt.

Diese obengenannten Verfahren sind Standardverfahren der Flüssigkristallchemie und dem Fachmann bekannt.

Die Verbindungen der Formel I können in Form von Gemischen untereinander und/oder mit anderen Flüssigkristallkomponenten verwendet werden. Geeignete Flüssigkristallkomponenten sind dem Fachmann in grosser Zahl bekannt, z.B. aus D. Demus et al., Flüssige Kristalle in Tabellen, VEB Deutscher Verlag für Grundstoffindustrie, Leipzig, Bände I und II, und viele davon sind zudem im Handel erhältlich.

Die Erfindung betrifft ebenfalls flüssigkristalline Gemische mit mindestens 2 Komponenten, welche dadurch gekennzeichnet sind, dass mindestens eine Komponente eine Verbindung der Formel I ist. Eine zweite und gegebenenfalls weitere Komponenten können weitere Verbindungen der allgemeinen Formel I oder andere geeignete Flüssigkristallkomponenten sein.

Aufgrund der guten Löslichkeit der erfindungsgemässen Verbindungen der Formel I in anderen Flüssigkristallmaterialien und aufgrund ihrer guten Mischbarkeit untereinander kann der Anteil an Verbindungen der Formel I in den erfindungsgemässen Gemischen relativ hoch sein und beispielsweise 1-40 Gew.-% betragen. Im allgemeinen ist ein Anteil von etwa 2-30 Gew.-%, insbesondere von etwa 3-25 Gew.-%, an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der Formeln: 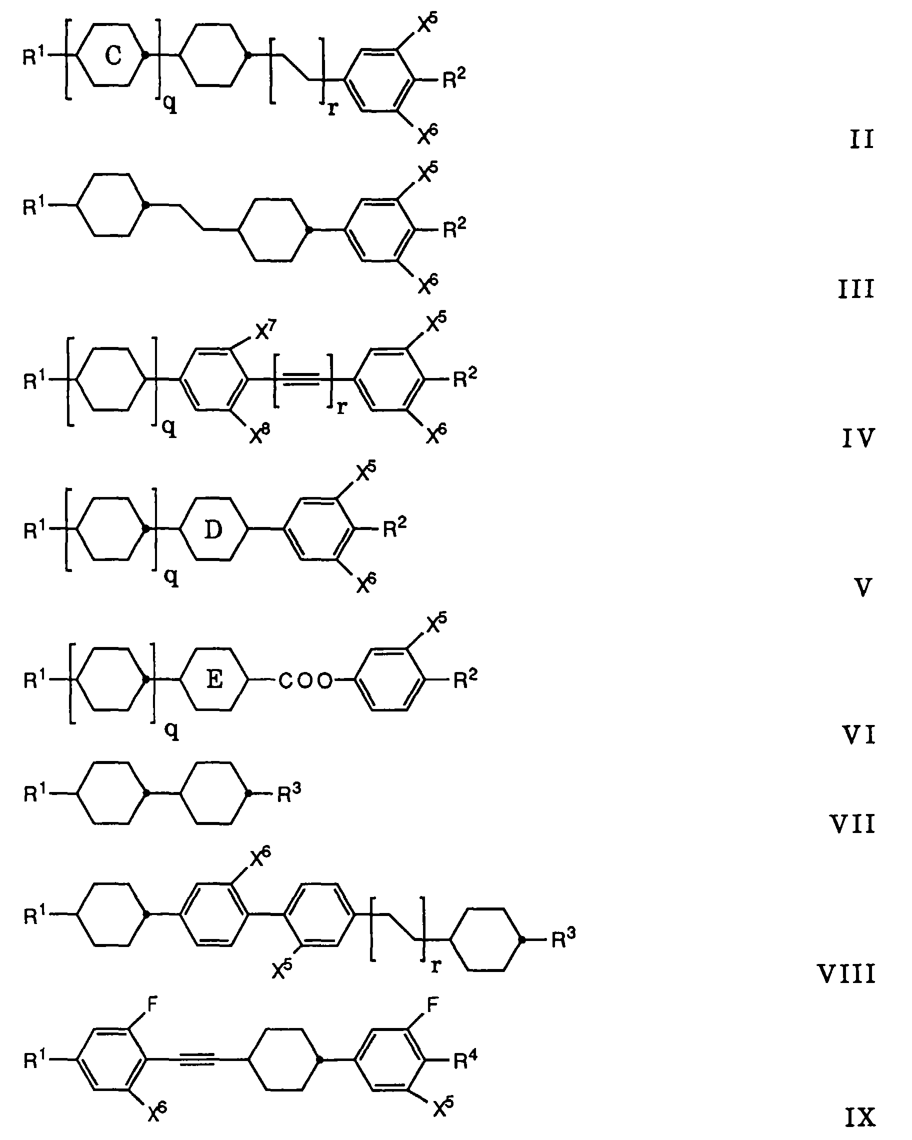 worin
- R¹ und R³: Alkyl, Alkoxyalkyl, 3E-Alkenyl, Alkenyl mit endständiger Doppelbindung, sowie an einem gesättigten Ring auch 1E-Alkenyl, mit jeweils höchstens 7 Kohlenstoffatomen;
- R²: Halogen, Cyano, -OCF₃, -OCHF₂, -CF₃, Alkyl, Alkoxy, Alkoxyalkyl, 2E-Alkenyloxy, 3E-Alkenyl oder Alkenyl oder Alkenyloxy mit endständiger Doppelbindung mit jeweils höchstens 5 Kohlenstoffatomen;
- R⁴: Halogen, Cyano, -OCF₃, -OCHF₂ oder -CF₃;
- X⁵-X⁸: unabhängig voneinander Wasserstoff oder Fluor;
- Ring C: trans-1,4-Cyclohexylen oder trans-1,3-Dioxan-5,2-diyl;
- Ring D: trans-1,3-Dioxan-5,2-diyl, Pyridin-5,2-diyl oder Pyrimidin-5,2-diyl;
- Ring E: trans-1,4-Cyclohexylen oder gegebenenfalls mit Fluor mono- oder di-substituiertes 1,4-Phenylen; und
- q und r: unabhängig voneinander 0 oder 1 bedeuten.

Die im Zusammenhang mit den Verbindungen der Formeln II bis IX verwendeten Ausdrücke sind wie weiter vorne definiert.
Der Ausdruck "gesättigte Ringe" bezeichnet trans-1,4-Cyclohexylen oder trans-1,3-Dioxan-2,5-diyl.
Der Ausdruck "Halogen" bedeutet im Zusammenhang mit den Formeln II-IX Fluor oder Chlor.
Der Ausdruck "Alkyl mit höchstens 7 Kohlenstoffatomen" umfasst geradkettige Reste, wie Methyl, Ethyl, Propyl, Butyl, Pentyl, Hexyl und Heptyl und dergleichen.
Der Ausdruck "Alkoxylalkyl mit höchstens 7 Kohlenstoffatomen" umfasst geradkettige Reste, wie Methoxymethyl, Methoxyethyl, Methoxypropyl, Methoxybutyl, Methoxypentyl, Methoxyhexyl, Ethoxymethyl, Ethoxyethyl, Ethoxypropyl und dergleichen.
Der Ausdruck "3E-Alkenyl mit höchstens 7 Kohlenstoffatomen" umfasst 3E-Pentenyl, 3E-Hexenyl und 3E-Heptenyl.
Der Ausdruck "Alkenyl mit endständiger Doppelbindung mit höchstens 7 Kohlenstoffatomen" umfasst 2-Propenyl, 3-Butenyl, 4-Pentenyl, 5-Hexenyl, 6-Heptenyl und an einem gesättigten Ring auch Vinyl.
Der Ausdruck "1E-Alkenyl mit höchstens 7 Kohlenstoffatomen" umfasst 1E-Propenyl, 1E-Butenyl, 1E-Pentenyl, 1E-Hexenyl und 1E-Heptenyl. Der Ausdruck "Alkoxy" umfasst geradkettige Reste, worin Alkyl wie oben definiert ist.
Der Ausdruck "Alkenyloxy" mit endständiger Doppelbindung umfasst Reste, worin Alkenyl wie oben definiert ist.

Bevorzugt werden Reste R¹ und R³ mit jeweils höchstens 5 Kohlenstoffatomen.

Die erfindungsgemässen Mischungen können ferner optisch aktive Verbindungen (z.B. optisch aktive 4'-Alkyl- oder 4'-Alkoxy-4-biphenylcarbonitrile) enthalten. Der Anteil solcher Verbindungen wird durch die Löslichkeit, die gewünschte Ganghöhe der Helix, und dergleichen bestimmt. Im allgemeinen beträgt der Anteil optisch aktiver Verbindungen höchstens etwa 5 Gew.% im Gesamtgemisch.

Ebenfalls Gegenstand der vorliegenden Erfindung sind elektro-optische Anzeigevorrichtungen enthaltend die obengenannten, erfindungsgemässen flüssigkristallinen Gemische.

Die Herstellung der flüssigkristallinen Gemische und der elektro-optischen Anzeigevorrichtung kann in an sich bekannter Weise erfolgen.

Die Herstellung der Verbindungen der Formel I sowie flüssigkristalliner Gemische enthaltend diese Verbindungen werden durch die folgenden Beispiele weiter veranschaulicht. C bedeutet eine kristalline, S eine smektische, N die nematische und I die isotrope Phase. V₁₀ bezeichnet die Spannung für 10% Transmission (Blickrichtung senkrecht zur Plattenoberfläche). tₒₙ und t_{off} bezeichnen die Einschaltzeit bzw. die Ausschaltzeit, Δn die optische Anisotropie, und GC bedeutet Gaschromatographie.

### Beispiel 1

Eine Lösung von 0,40 g 4-Cyclopentyliden-trans-4'-(3,4-difluorphenyl)-bicyclohexyl in 15 ml Toluol/Ethanol 1:2 (v/v) wurde mit 50 mg Platin(IV)oxid bei Raumtemperatur und Normaldruck bis zum Stillstand der Wasserstoffaufnahme hydriert. Das nach Filtration und Einengen des Filtrats erhaltene Rohprodukt wurde dreimal aus Ethanol umkristallisiert. Es wurden 0,184 g reines trans-4-Cyclopentyl-trans-4'-(3,4-difluorphenyl)-bicyclohexyl erhalten; Smp. (C-S_{B}) 97,8°C, S_{B}-N 101,4°C, Klp. (N-I) 145°C.

Das als Ausgangsmaterial verwendete 4-Cyclopentyliden-trans-4'-(3,4-difluorphenyl)-bicyclohexyl wurde wie folgt hergestellt:

Eine Suspension von 5,6 g Cyclopentyl-triphenyl-phosphoniumbromid in 50 ml t-Butyl-methylether wurde mit 1,4 g Kalium-t-butylat versetzt. Die entstehende rote Suspension wurde 45 Minuten bei Raumtemperatur gerührt und dann bei 3°C tropfenweise mit einer Lösung von 0,80 g 4-[trans-4-(3,4-Difluorphenyl)-cyclohexyl]-cyclohexanon in 20 ml t-Butyl-methylether versetzt. Das Reaktionsgemisch wurde noch 15 Minuten bei 3°C und 6 Stunden bei Raumtemperatur gerührt, dann mit 25 ml gesättigter Natriumhydrogencarbonat-Lösung gerührt und mit Diethylether verdünnt. Die Wasserphase wurde abgetrennt und mit Diethylether extrahiert. Die organische Phase wurde vereinigt, mit Wasser neutral gewaschen, über Natriumsulfat getrocknet, filtriert und das Filtrat eingeengt. Der Rückstand wurde in 30 ml Methanol/Wasser 4:1 (v/v) und Hexan gelöst. Die Methanol-Phase wurde abgetrennt und die Hexan-Phase je dreimal mit 15 ml Methanol/Wasser 4:1 gewaschen und eingeengt. Der Rückstand (1,9 g) wurde an 40 g Kieselgel mit Hexan flashchromatographiert. Von den produkthaltigen Fraktionen (0,80 g; GC-Reinheit 77%) wurde im Kugelrohr bei 180-200°/ca. 0,5 Torr ein leicht-flüchtiges Nebenprodukt (0,17 g) abdestilliert.

Der Destillationsrückstand (0,49 g, GC-Reinheit 99 %) wurde zweimal aus je 10 ml Ethanol umkristallisiert. Es resultierten 0,43 g reines 4-Cyclopentyliden-trans-4'-(3,4-difluorphenyl)-bicyclohexan; Smp. 104,4 °C.

In analoger Weise können hergestellt werden:
trans-4-Cyclopentyl-trans-4'-(3,4,5-trifluorphenyl)-bicyclohexyl;
trans-4-Cyclopentyl-trans-4'-(4-trifluormethoxy-phenyl)-bicyclohexyl;
trans-4-Cyclopentyl-trans-4'-(4-trifluormethoxy-3-fluorphenyl)-bicyclohexyl;
trans-4-Cyclopentyl-trans-4'-(4-difluormethoxy-phenyl)-bicyclohexyl;
trans-4-Cyclopentyl-trans-4'-(4-difluormethoxy-3-fluorphenyl)-bicyclohexyl;
trans-4-Cyclopentyl-trans-4'-(4-difluormethoxy-3,5-difluorphenyl)-bicyclohexyl;
trans-4-Cyclopentyl-trans-4'-(3,4-difluorphenethyl)-bicyclohexyl;
trans-4-Cyclopentyl-trans-4'-(3,4,5-trifluorphenethyl)-bicyclohexyl;
trans-4-Cyclopentyl-trans-4'-(4-trifluormethoxy-phenethyl)-bicyclohexyl;
trans-4-Cyclopentyl-trans-4'-(4-difluormethoxy-3-fluorphenethyl)-bicyclohexyl;
trans-4-Cyclopentyl-trans-4'-(4-difluormethoxy-3,5-difluorphenethyl)-bicyclohexyl;
1-[trans-4-[2-(trans-4-Cyclopentyl-cyclohexyl)-ethyl]-cyclohexyl]-3,4-difluorbenzol;
1-[trans-4-[2-(trans-4-Cyclopentyl-cyclohexyl)-ethyl]-cyclohexyl]-3,4,5-trifluorbenzol;
1-[trans-4-[2-(trans-4-Cyclopentyl-cyclohexyl)-ethyl]-cyclohexyl]-4-trifluormethoxybenzol;
1-[trans-4-[2-(trans-4-Cyclopentyl-cyclohexyl)-ethyl]-cyclohexyl]-4-difluormethoxy-3-fluorbenzol;
1-[trans-4-[2-(trans-4-Cyclopentyl-cyclohexyl)-ethyl]-cyclohexyl]-4-difluormethoxy-3,5-difluorbenzol;
trans-4'-(4-Cyclopentyl-cyclohexyl)-3,4-difluorbiphenyl;
trans-4'-(4-Cyclopentyl-cyclohexyl)-3,4,5-trifluorbiphenyl;
trans-4'-(4-Cyclopentyl-cyclohexyl)-3,4',2'-trifluorbiphenyl;
trans-4'-(4-Cyclopentyl-cyclohexyl)-4-trifluormethoxy-biphenyl;
trans-4'-(4-Cyclopentyl-cyclohexyl)-2'-fluor-4-trifluormethoxy-biphenyl;
trans-4'-(4-Cyclopentyl-cyclohexyl)-4-difluormethoxy-3-fluorbiphenyl;
trans-4'-(4-Cyclopentyl-cyclohexyl)-4-difluormethoxy-3,5-difluorbiphenyl;
trans-4'-[2-(4-Cyclopentyl-cyclohexyl)-ethyl]-3,4-difluorbiphenyl;
trans-4'-[2-(4-Cyclopentyl-cyclohexyl)-ethyl]-4-trifluormethoxy-biphenyl;
trans-4'-[2-(4-Cyclopentyl-cyclohexyl)-ethyl]-4-difluormethoxy-3-fluorbiphenyl;
4-(trans-4'-Cyclopentyl-bicyclohexyl-trans-4-yl)-benzonitril;
4-(trans-4'-Cyclopentyl-bicyclohexyl-trans-4-yl)-2-fluorbenzonitril;
4-[2-(trans-4'-Cyclopentyl-bicyclohexyl-trans-4-yl)-ethyl]-2-fluorbenzonitril;
4'-(trans-4-Cyclopentyl-cyclohexyl)-biphenyl-4-carbonitril;
trans-4-(Cyclopentyl-methyl)-trans-4'-(3,4-difluorphenyl)-bicyclohexyl, Smp. (C-N) 76,4 °C, S_{B}-N 40,5°C, Klp. (N-I) 95,5 °C;
trans-4-(Cyclopentyl-methyl)-trans-4'-(3,4,5-trifluorphenyl)-bicyclohexyl;
trans-4-(Cyclopentyl-methyl)-trans-4'-(4-trifluormethoxy-phenyl)-bicyclohexyl;
trans-4-(Cyclopentyl-methyl)-trans-4'-(4-difluormethoxy-3-fluorphenyl)-bicyclohexyl;
trans-4-(Cyclopentyl-methyl)-trans-4'-(4-difluormethoxy-3,5-difluorphenyl)-bicyclohexyl;
trans-4-(Cyclopentyl-methyl)-trans-4'-(3,4-difluorphenethyl)-bicyclohexyl;
trans-4-(Cyclopentyl-methyl)-trans-4'-(3,4,5-trifluorphenethyl)-bicyclohexyl;
trans-4-(Cyclopentyl-methyl)-trans-4'-(4-trifluormethoxy-phenethyl)-bicyclohexyl;
trans-4-(Cyclopentyl-methyl)-trans-4'-(4-difluormethoxy-3-fluorphenethyl)-bicyclohexyl;
1-[trans-4-[2-[trans-4-(Cyclopentyl-methyl)-cyclohexyl]-ethyl]-cyclohexyl]-3,4,5-trifluorbenzol;
trans-4'-[4-(Cyclopentyl-methyl)-cyclohexyl]-3,4-difluorbiphenyl;
trans-4'-[4-(Cyclopentyl-methyl)-cyclohexyl]-3,4,5-trifluorbiphenyl;
trans-4'-[2-[4-(Cyclopentyl-methyl)-cyclohexyl]-ethyl]-3,4,5-trifluorbiphenyl.

### Beispiel 2

Eine Lösung von 0.54 g trans-4-(2-Cyclopentyl-vinyl)-trans-4'-(3,4,5-trifluorphenyl)-bicyclohexyl in 12 ml Toluol wurde mit 80 mg 5% Palladium auf Kohle bei Raumtemperatur und Normaldruck bis zum Stillstand hydriert. Das nach Filtration und Einengen des Filtrats erhaltene Rohprodukt wurde mehrmals aus Ethanol umkristallisiert. Es wurde reines trans-4-(2-Cyclopentylethyl)-trans-4'-(3,4,5-trifluorphenyl)-bicyclohexyl erhalten; Smp. (C-N) 91,6 °C, Klp. (N-I) 99,6 °C.

Das als Ausgangsmaterial verwendete trans-4-(2-Cyclopentyl-vinyl)-trans-4'-(3,4,5-trifluorphenyl)-bicyclohexyl wurde wie folgt hergestellt:

Eine Suspension von 2,3 g (Cyclopentyl-ethyl)-triphenyl-phosphoniumbromid in 15 ml t-Butylmethylether wurde mit 0,59 g Kalium-t-butylat versetzt. Die entstehende orange Suspension wurde 45 Minuten bei Raumtemperatur gerührt und dann bei 3 °C tropfenweise mit einer Lösung von 0,85 g trans-4'-(3,4,5-trifluorphenyl)-trans-4-bicyclohexyl-carboxaldehyd in 15 ml t-Butyl-methylether versetzt. Das Reaktionsgemisch wurde noch 15 Minuten bei 3 °C und 2 Std. bei Raumtemperatur gerührt, dann mit 30 ml gesättigier Natriumhydrogencarbonat-Lösung verrührt. Die wässrige Phase wurde abgetrennt und mit Diethylether extrahiert. Die organischen Phasen wurden vereinigt, mit Wasser neutral gewaschen, über Natriumsulfat getrocknet, filtriert und das Filtrat eingeengt. Der Rückstand wurde in Methanol/Wasser 4:1 und Hexan aufgenommen. Die Methanol-Phase wurde abgetrennt und die Hexan-Phase zweimal mit je 20 ml Methanol/Wasser 4:1 gewaschen und anschliessend eingeengt. Flashchromatographie des Rückstands an 35 g Kieselgel mit Hexan ergab 0,74 g trans-4-(2-Cyclopentylvinyl)-trans-4'-(3,4,5-trifluorphenyl)-bicyclohexyl.

In analoger Weise können folgende Verbindungen hergestellt werden:
trans-4-(3-Cyclopentyl-propyl)-trans-4'-(3,4,5-trifluorphenyl)-bicyclohexyl;
trans-4-(4-Cyclopentyl-butyl)-trans-4'-(3,4,5-trifluorphenyl)-bicyclohexyl;
trans-4-(5-Cyclopentyl-pentyl)-trans-4'-(3,4,5-trifluorphenyl)-bicyclohexyl;
trans-4-(2-Cyclopentyl-ethyl)-trans-4'-(3,5-difluor-4-difluormethoxyphenyl)-bicyclohexyl;
trans-4-(3-Cyclopentyl-propyl)-trans-4'-(3,5-difluor-4-difluormethoxyphenyl)-bicyclohexyl;
trans-4-(4-Cyclopentyl-butyl)-trans-4'-(3,5-difluor-4-difluormethoxyphenyl)-bicyclohexyl;
trans-4-(2-Cyclopentyl-ethyl)-trans-4'-(4-cyano-3,5-difluorphenyl)-bicyclohexyl;
trans-4-(3-Cyclopentyl-propyl)-trans-4'-(4-cyano-3,5-difluorphenyl)-bicyclohexyl;
trans-4-(4-Cyclopentyl-butyl)-trans-4'-(4-cyano-3,5-difluorphenyl)-bicyclohexyl;
trans-4-(2-Cyclopentyl-ethyl)-trans-4'-[3,5-difluor-4-(2,2,2-trifluorethoxy)phenyl]-bicyclohexyl;
trans-4-(2-Cyclopentyl-ethyl)-trans-4'-(3,4-difluor-phenyl)-bicyclohexyl, Smp. (C-S_{B}) 57 °C, S_{B}-N 74,8 °C, Klp. (N-I) 126,3 °C;
trans-4-(3-Cyclopentyl-propyl)-trans-4'-(3,4-difluor-phenyl)-bicyclohexyl, Smp. (C-N) 54,0 °C, S_{B}-N 19,1 °C, Klp. (N-I) 99,5 °C;
trans-4-(4-Cyclopentyl-butyl)-trans-4'-(3,4-difluor-phenyl)-bicyclohexyl, Smp. (C-N) 61,1°C, S_{B}-N 59,8 °C, Klp. (N-I) 116,0 °C;
trans-4-(2-Cyclopentyl-ethyl)-trans-4'-(4-chlor-3-fluor-phenyl)-bicyclohexyl;
trans-4-(2-Cyclopentyl-ethyl)-trans-4'-(4-(difluormethoxy)-3-fluorophenyl]-bicyclohexyl;
trans-4-(3-Cyclopentyl-propyl)-trans-4'-(4-difluormethoxyphenyl)-bicyclohexyl;
trans-4-(4-Cyclopentyl-butyl)-trans-4'-(4-difluormethoxyphenyl)-bicyclohexyl;
trans-4-(2-Cyclopentyl-ethyl)-trans-4'-(3-fluor-4-trifluormethoxy-phenyl)-bicyclohexyl;
trans-4-(2-Cyclopentyl-butyl)-trans-4'-(3-fluor-4-trifluormethoxy-phenyl)-bicyclohexyl;
trans-4-(2-Cyclopentylethyl)-trans-4'-(4-cyano-3-fluorphenyl)-bicyclohexyl;
trans-4-(3-Cyclopentyl-propyl)-trans-4'-(4-cyano-3-fluor-phenyl)-bicyclohexyl;
trans-4-(4-Cyclopentyl-butyl)-trans-4'-(4-cyano-3-fluorphenyl)-bicyclohexyl;
trans-4-(2-Cyclopentyl-ethyl)-trans-4'-(4-trifluormethoxy-phenyl)-bicyclohexyl;
trans-4-(3-Cyclopentyl-propyl)-trans-4'-(4-trifluormethoxyphenyl)-bicyclohexyl;
trans-4-(4-Cyclopentyl-butyl)-trans-4'-(4-trifluormethoxyphenyl)-bicyclohexyl;
trans-4-(5-Cyclopentyl-pentyl)-trans-4'-(4-trifluormethoxyphenyl)-bicyclohexyl,;
trans-4-(2-Cyclopentyl-ethyl)-trans-4'-(4-difluormethoxy-phenyl)-bicyclohexyl;
trans-4-(2-Cyclopentyl-ethyl)-trans-4'-(4-trifluormethyl-phenyl)-bicyclohexyl;
trans-4-(2-Cyclopentyl-ethyl)-trans-4'-(4-fluorphenyl)-bicyclohexyl;
trans-4-(3-Cyclopentyl-propyl)-trans-4'-(4-fluor-phenyl)-bicyclohexyl;
trans-4-(4-Cyclopentyl-pentyl)-trans-4'-(4-fluor-phenyl)-bicyclohexyl;
trans-4-(2-Cyclopentyl-ethyl)-trans-4'-(4-chlorphenyl)-bicyclohexyl;
trans-4-(2-Cyclopentyl-ethyl)-trans-4'-(4-cyano-phenyl)-bicyclohexyl;
4'-[trans-4-(2-Cyclopentyl-ethyl)-cyclohexyl]-3,4,5-trifluor-biphenyl;
4'-[trans-4-(3-Cyclopentyl-propyl)-cyclohexyl]-3,4,5-trifluor-biphenyl;
4'-[trans-4-(4-Cyclopentyl-butyl)-cyclohexyl]-3,4,5-trifluor-biphenyl;
4'-[trans-4-(2-Cyclopentyl-ethyl)-cyclohexyl]-3,4,2'-trifluor-biphenyl;
4'-[trans-4-(3-Cyclopentyl-propyl)-cyclohexyl]-3,4,2'-trifluor-biphenyl;
4'-[trans-4-(4-Cyclopentyl-butyl)-cyclohexyl]-3,4,2'-trifluor-biphenyl;
4'-[trans-4-(2-Cyclopentyl-ethyl)-cyclohexyl]-4,2',6'-trifluor-biphenyl;
4-[trans-4-(2-Cyclopentyl-ethyl)-cyclohexyl]-3,4,2',6'-tetrafluor-biphenyl;
4-[trans-4-(2-Cyclopentyl-ethyl)-cyclohexyl]-4-difluormethoxy-3,5-difluorbiphenyl;
4-[trans-4-(3-Cyclopentyl-propyl)-cyclohexyl]-4-difluormethoxy-3,5-difluor-biphenyl;
4-[trans-4-(4-Cyclopentyl-butyl)-cyclohexyl]-4-difluormethoxy-3,5-difluorbiphenyl;
4-[trans-4-(2-Cyclopentyl-ethyl)-cyclohexyl]-4-difluormethoxy-3,2'-difluor-biphenyl;
4'-[trans-4-(2-Cyclopentyl-ethyl)-cyclohexyl]-4-trifluormethoxy-3,2'-difluor-biphenyl;
4'-[trans-4-(2-Cyclopentyl-ethyl)-cyclohexyl]-4-trifluormethoxy-2',6'-difluor-biphenyl;
4'-[trans-4-(3-Cyclopentyl-propyl)-cyclohexyl]-4-trifluormethoxy-2',6'-difluor-biphenyl;
4'-[trans-4-(4-Cyclopentyl-butyl)-cyclohexyl]-4-trifluormethoxy-2',6'-difluor-biphenyl;
4'-[trans-4-(2-Cyclopentyl-ethyl)-cyclohexyl]-4-trifluormethoxy-2',6'-difluor-biphenyl;
4'-(4-Cyclopentyl-butyl)-3,4-difluorbiphenyl;
4'-(5-Cyclopentyl-pentyl)-3,4-difluorbiphenyl;
4'-(2-Cyclopentyl-ethyl)-3,4-difluortolan;
4'-(4-Cyclopentyl-butyl)-3,4-difluortolan;
4'-(4-Cyclopentyl-butyl)-4-trifluormethoxy-tolan;
5-(4-Cyclopentyl-butyl)-2-(3,4-difluorphenyl)-pyridin;
5-(4-Cyclopentyl-butyl)-2-(3,4-difluorphenyl)-pyrimidin;
5-(5-Cyclopentyl-pentyl)-2-(3,4-difluorphenyl)-pyrimidin;
5-(6-Cyclopentyl-hexyl)-2-(3,4-difluorphenyl)-pyrimidin;
5-(7-Cyclopentyl-heptyl)-2-(3,4-difluorphenyl)-pyrimidin;
5-(4-Cyclopentyl-butyl)-2-(4-trifluormethoxy-phenyl)-pyrimidin;
5-(6-Cyclopentyl-hexyl)-2-(4-trifluormethoxy-phenyl)-pyrimidin;
5-(4-Cyclopentyl-butyl)-2-(3-fluor-4-cyanophenyl)-pyrimidin;
5-(5-Cyclopentyl-pentyl)-2-(3-fluor-4-cyanophenyl)-pyrimidin;
5-(6-Cyclopentyl-hexyl)-2-(3-fluor-4-cyanophenyl)-pyrimidin;
trans-1-(4-Cyclopentyl-butyl)-4-(4-trifluormethoxy-phenyl)-cyclohexan;
trans-1-(6-Cyclopentyl-hexyl)-4-(4-trifluormethoxy-phenyl)-cyclohexan;
trans-1-(2-Cyclopentyl-ethyl)-4-(3,4-difluorphenyl)-cyclohexan;
trans-1-(4-Cyclopentyl-butyl)-4-(3,4-difluorphenyl)-cyclohexan;
trans-1-(6-Cyclopentyl-hexyl)-4-(3,4-difluorphenyl)-cyclohexan;
trans-1-(6-Cyclopentyl-hexyl)-4-(4-chlor-3-fluorphenyl)-cyclohexan;
trans-1-(2-Cyclopentyl-ethyl)-4-(3-fluor-4-trifluormethoxy-phenyl)-cyclohexan;
trans-1-(4-Cyclopentyl-butyl)-4-(3-fluor-4-trifluormethoxy-phenyl)-cyclohexan;
trans-1-(6-Cyclopentyl-hexyl)-4-(3-fluor-4-trifluormethoxy-phenyl)-cyclohexan;
trans-1-(4-Cyclopentyl-butyl)-4-(4-difluormethyl-3-fluorphenyl)-cyclohexan;
trans-1-(6-Cyclopentyl-hexyl)-4-(4-difluormethyl-3-fluorphenyl)-cyclohexan;
trans-1-(2-Cyclopentyl-ethyl)-4-(3,4,5-trifluorphenyl)-cyclohexan;
trans-1-(4-Cyclopentyl-butyl)-4-(3,4,5-trifluorphenyl)-cyclohexan;
trans-1-(5-Cyclopentyl-pentyl)-4-(3,4,5-trifluorphenyl)-cyclohexan;
trans-1-(6-Cyclopentyl-hexyl)-4-(3,4,5-trifluorphenyl)-cyclohexan;
trans-1-(7-Cyclopentyl-heptyl)-4-(3,4,5-trifluorphenyl)-cyclohexan;
trans-1-(4-Cyclopentyl-butyl)-4-(3,5-difluor-4-trifluormethoxy-phenyl)-cyclohexan;
trans-1-(4-Cyclopentyl-butyl)-4-(3,5-difluor-4-difluormethoxy-phenyl)-cyclohexan;
trans-1-(6-Cyclopentyl-hexyl)-4-(3,5-difluor-4-difluormethoxy-phenyl)-cyclohexan;
trans-1-(4-Cyclopentyl-butyl)-4-(4-trifluormethoxy-phenethyl)-cyclohexan;
trans-1-(4-Cyclopentyl-butyl)-4-(3,4-difluor-phenethyl)-cyclohexan;
trans-1-(6-Cyclopentyl-hexyl)-4-(3,4-difluor-phenethyl)-cyclohexan;
trans-1-(4-Cyclopentyl-butyl)-4-(4-trifluormethoxy-3-fluor-phenethyl)-cyclohexan;
trans-1-(4-Cyclopentyl-butyl)-4-(4-difluormethoxy-3-fluor-phenethyl)-cyclohexan;
trans-1-(6-Cyclopentyl-hexyl)-4-(4-difluormethoxy-3-fluor-phenethyl)-cyclohexan;
trans-1-(4-Cyclopentyl-butyl)-4-(3,4,5-trifluor-phenethyl)-cyclohexan;
trans-1-(5-Cyclopentyl-pentyl)-4-(3,4,5-trifluor-phenethyl)-cyclohexan;
trans-1-(6-Cyclopentyl-hexyl)-4-(3,4,5-trifluor-phenethyl)-cyclohexan;
trans-1-(4-Cyclopentyl-butyl)-4-(4-trifluormethoxy-3,5-difluor-phenethyl)-cyclohexan;
trans-1-(4-Cyclopentyl-butyl)-4-(4-difluormethoxy-3,5-difluor-phenethyl)-cyclohexan;
trans-1-(6-Cyclopentyl-hexyl)-4-(4-difluormethoxy-3,5-difluor-phenethyl)-cyclohexan.

### Beispiel 3

Ein Gemisch aus 1,902 g 4-Cyclopentyl-benzoesäure, 1,371 g 2-Fluor-4-hydroxy-benzonitril, 0,122 g 4-Dimethylamino-pyridin und 50 ml Dichlormethan wurde innert 3 Minuten portionenweise mit 2,37 g N,N'-Dicyclohexyl-carbodiimid versetzt. Die entstandene Suspension wurde 75 Minuten bei Raumtemperatur gerührt und filtriert. Das Filtrat wurde mit 150 ml Dietylether verdünnt. Die Lösung wurde mit 45 ml 2 M Salzsäure, 20 ml Wasser, 15 ml 1 M Soda-Lösung und zweimal je 20 ml Wasser gewaschen über Natriumsulfat getrocknet, filtriert und das Filtrat eingeengt. Das Rohprodukt wurde an 23 g Kieselgel mit Hexan/Ethylacetat 23 : 2 (v/v) chromatographiert. Zweimalige Umkristallisation der produkthaltigen Fraktionen (2,254 g) ergab 1,982 g reinen Cyclopentylbenzosäure-4-cyano-3-fluor-phenylester; Smp. (C-I) 77,2 °C, Klp. (N-I) 27,3°C.

Auf analoge Weise können folgende Produkte hergestellt werden:
4-Cyclopentylmethyl-benzoesäure-4-cyano-3-fluorphenylester, Smp. (C-I) 54,2 °C;
4-(2-Cyclopentyl-ethyl)-benzoesäure-4-cyano-3-fluorphenylester, Smp. (C-I) 43,2 °C, Klp. (N-I) 24,1 °C;
4-(3-Cyclopentyl-propyl)-benzoesäure-4-cyano-3-fluorphenylester, Smp. (C-I) 50,6 °C, Klp. (N-I) -4,9 °C;
4-(4-Cyclopentyl-butyl)-benzoesäure-4-cyano-3-fluorphenylester, Smp. (C-I) 50,1 °C, Klp. (N-I) 24,9 °C;
4-(5-Cyclopentyl-pentyl)-benzoesäure-4-cyano-3-fluorphenylester;
4-(Cyclopentyl-methoxy)-benzoesäure-4-cyano-3-fluorphenylester, Smp. (C-I) 63,3°C, Klp. (N-I) 20,7°C;
4-(3-Cyclopentyl-propyloxy)-benzoesäure-4-cyano-3-fluorphenylester;
4-Cyclopentyl-2-fluorbenzoesäure-4-cyano-3-fluorphenylester;
4-(2-Cyclopentyl-ethyl)-2-fluorbenzoesäure-4-cyano-3-fluorphenylester;
4-Cyclopentyl-benzoesäure-4-cyano-3,5-difluorpheylester;
4-(2-Cyclopentyl-ethyl)-benzoesäure-4-cyano-3,5-difluorphenylester;
4-Cyclopentyl-benzoesäure-3,4-difluorphenylester.

### Beispiel 4

Ein Gemisch aus 340 mg 1-Cyclopentyl-4-ethinylbenzol, 430 mg 5-Brom-1,2,3-trifluorbenzol, 47 mg Tetrakis(triphenylphosphin)palladium, 17 mg Kupfer(I)bromid und 12 ml Triethylamin werden über Nacht zum Sieden erhitzt. Das Reaktionsgemisch wird im Vakuum eingedampft. Der Rückstand wird in 30 ml Diethylether und 10 ml Wasser aufgenommen. Die Etherphase wird mit Wasser gewaschen, über Natriumsulfat getrocknet, filtriert und das Filtrat eingeengt. Chromatographie des Rückstands an 10 g Kieselgel mit Hexan liefert reines 4'-Cyclopentyl-3,4,5-trifluortolan.

Das als Ausgangsmaterial verwendete 1-Cyclopentyl-4-ethinylbenzol kann wie folgt hergestellt werden:
a) Eine Lösung von 13,1 g Triphenylphosphin in 30 ml Dichlormethan wird innert 5 Minuten bei 5-10°C zu einer Lösung von 8,3 g Tetrabrommethan in 30 ml Dichlormethan getropft. Die orange Lösung wird noch 10 Min. bei 5° C gerührt, dann wird bei der gleichen Temperatur innert 10 Minuten eine Lösung von 8,7 g 4-Cyclopentylbenzaldehyd in 20 ml Dichlormethan zugetropft. Die Lösung wird noch 30 Min. bei 5 °C gerührt, dann mit 2 x 30 ml gesättigter Natriumhydrogencarbonat-Lösung vorsichtig extrahiert, bis die CO₂-Entwicklung beendet ist, mit 50 ml Wasser gewaschen, über Natriumsulfat getrocknet, filtriert und das Filtrat eingeengt. Der Rückstand wird in je 50 ml Hexan und 80%igem wässrigen Methanol aufgenommen. Die Hexanphase wird mit 20 ml 80%igem Methanol gewaschen und dann eingeengt. Das erhaltene 1-Cyclopentyl-4-(2,2-dibromvinyl)-benzol wird in 50 ml Tetrahydrofuran gelöst. Ca. 10 ml dieser Lösung werden zu 0.42 g Magnesiumspänen gegeben. Das Magnesium wird durch Zugabe von wenig Brom aktiviert und die Lösung erhitzt, bis die Reaktion einsetzt, dann wird die restliche Lösung so zugetropft, dass das Gemisch leicht siedet. Danach wird noch 1 Stunde zum Sieden erhitzt, mit 50 ml Hexan versetzt und mit 30 ml halbgesättigtem Natrumhydogencarbonat-Lösung und 30 ml Wasser gewaschen. Die organische Phase wird über Natriumsulfat getrocknet, filtriert und das Filtrat eingeengt. Destillation des Rückstandes liefert 1-Cyclopentyl-4-ethinyl-benzol.

Analog können folgende Verbindungen hergestellt werden:
4'-Cyclopentyl-4-trifluormethyl-tolan
4'-Cyclopentyl-4-trifluormethoxy-tolan
4'-Cyclopentyl-3,4-difluortolan
4'-Cyclopentyl-4-difluormethoxy-3-fluortolan
4'-(2-Cyclopentyl-ethyl)-3,4-difluortolan
4'-(trans-4-Cyclopentyl-cyclohexyl)-3,4-difluor-tolan
4'-(trans-4-Cyclopentyl-cyclohexyl)-4-trifluormethoxy-tolan
4'-(trans-4-Cyclopentyl-cyclohexyl)-3,4,5-trifluortolan
4'-(trans-4-Cyclopentyl-cyclohexyl)-3,4,2'-trifluortolan
4'-(trans-4-Cyclopentyl-cyclohexyl)-3,4,2',6'-tetrafluortolan
4'[trans-4-(2-Cyclopentyl-ethyl)cyclohexyl]-3,4-difluortolan
4'[trans-4-(2-Cyclopentyl-ethyl)cyclohexyl]-3,4,5-trifluortolan
4'[trans-4-(2-Cyclopentyl-ethyl)cyclohexyl]-3,4,2'-trifluortolan
4'[trans-4-(2-Cyclopentyl-ethyl)cyclohexyl]-3,4,2',6'-tetrafluortolan
4'[trans-4-(2-Cyclopentyl-ethyl)cyclohexyl]-3,4,5,2',6'-pentafluortolan

### Beispiel 5

Ein Gemisch aus 0,67 g trans-4-(3,4-Difluorphenyl)-cyclohexancarboxaldehyd, 0,46 g 2-Cyclopentyl-1,3-propandiol, 20 ml Toluol und 3 Tropfen 10%iger Schwefelsäure wird eine Stunde zum Sieden erhitzt, wobei feuchtes Toluol abdestilliert und durch frisches Toluol ersetzt wird. Das Reaktionsgemisch wird mit 2 Tropfen Triethylamin neutralisiert und nach dem Erkalten mit 5 ml Kochsalz-Lösung gewaschen, über Natriumsulfat getrocknet, filtriert und eingeengt. Der Rückstand wird an 15 g Kieselgel mit Hexan/Ethylacetat vol. 97:3 chromatographiert. Zweimalige Umkristallisation der produkthaltigen Fraktionen aus Ethanol liefert reines trans-5-Cyclopentyl-2-[trans-4-(3,4-difluorphenyl)-cyclohexyl]-1,3-dioxan.

Analog können folgende Verbindungen hergestellt werden:
trans-5-Cyclopentyl-2-[trans-4-(3,4,5-trifluorphenyl)-cyclohexyl]-1,3-dioxan,
trans-5-Cyclopentyl-2-[trans-4-(4-difluormethoxy-3,5-difluorphenyl)-cyclohexyl]-1,3-dioxan,
trans-5-Cyclopentyl-2-[trans-4-(4-trifluormethoxy-phenyl)-cyclohexyl]-1,3-dioxan,
trans-5-Cyclopentyl-2-[trans-4-(3-fluor-4-trifluormethoxy-phenyl)-cyclohexyl]-1,3-dioxan,
trans-5-Cyclopentyl-2-[trans-4-(3,4-difluorphenethyl)-cyclohexyl]-1,3-dioxan,
trans-5-Cyclopentyl-2-[trans-4-(3,4,5-trifluorphenethyl)-cyclohexyl]-1,3-dioxan,
trans-5-Cyclopentyl-2-[trans-4-(4-difluormethoxy-3-fluorphenethyl)-cyclohexyl]-1,3-dioxan,
trans-5-Cyclopentyl-2-[trans-4-(4-difluormethoxy-3,5-difluorphenethyl)-cyclohexyl]-1,3-dioxan,
trans-5-(2-Cyclopentyl-ethyl)-2-[trans-4-(3,4,5-trifluorphenyl)-cyclohexyl]-1,3-dioxan,
trans-5-(2-Cyclopentyl-ethyl)-2-[trans-4-(3,4,5-trifluorphenethyl)-cyclohexyl]-1,3-dioxan.

### Beispiel 6

Ein Gemisch aus 412 mg 3',4'-Difluor-biphenyl-4-ol, 420 mg (3-Brompropyl)-cyclopentan, 5 ml Dimethylformamid und 550 mg fein pulverisiertes Kaliumcarbonat wird 4 Stunden bei 50°C gerührt. Die Suspension wird mit 15 ml Toluol und 15 ml Wasser versetzt. Die Wasserphase wird abgetrennt. Die Toluol-Phase wird mehrmals mit Wasser gewaschen, über Natriumsulfat getrocknet, filtriert und das Filtrat eingeengt. Das Rohprodukt wird an 10 g Kieselgel mit Hexan/Ethylacetat vol. 49:1 chromatographiert. Umkristallisation aus Ethanol liefert reines 4'-(3-Cyclopentyl-propoxy)-3,4-difluorbiphenyl.

In analoger Weise können hergestellt werden:
4'-(5-Cyclopentyl-pentoxy)-3,4-difluorbiphenyl,
4'-(5-Cyclopentyl-pentoxy)-3,4,5-trifluorbiphenyl,
4'-(5-Cyclopentyl-pentoxy)-4-trifluormethoxy-biphenyl,
5-(5-Cyclopentyl-pentoxy)-2-(3,4-difluorphenyl)-pyridin,
5-(5-Cyclopentyl-pentoxy)-2-(3,4-difluorphenyl)-pyrimidin,
5-(6-Cyclopentyl-hexyloxy)-2-(3,4-difluorphenyl)-pyrimidin,
5-(7-Cyclopentyl-heptyloxy)-2-(3,4-difluorphenyl)-pyrimidin,
5-(5-Cyclopentyl-pentoxy)-2-(3,4,5-trifluorphenyl)-pyrimidin,
5-(5-Cyclopentyl-pentoxy)-2-(4-trifluormethoxy-phenyl)-pyrimidin,
5-(5-Cyclopentyl-pentoxy)-2-(4-difluormethoxy-3-fluorphenyl)-pyrimidin,
5-(5-Cyclopentyl-pentoxy)-2-(4-difluormethoxy-3,5-difluorphenyl)-pyrimidin,
4'-(Cyclopentyl-methoxy)-3,4-difluortolan,
4'-(Cyclopentyl-methoxy)-4-chlor-3-fluortolan,
4'-(Cyclopentyl-methoxy)-4-trifluormethoxy-tolan,
4'-(Cyclopentyl-methoxy)-4-difluormethoxy-3-fluor-tolan,
4'-(3-Cyclopropyl-propoxy)-3,4-difluortolan,
4'-(3-Cyclopropyl-propoxy)-4-chlor-3-fluor-tolan,
4'-(3-Cyclopropyl-propoxy)-4-trifluormethoxy-tolan,
4'-(3-Cyclopropyl-propoxy)-4-difluormethoxy-3-fluor-tolan,
4'-(3-Cyclopropyl-propoxy)-3,4,5-trifluor-tolan.

### Beispiel 7

Zur Untersuchung der Eigenschaften der Verbindungen der Formel I in Gemischen wurden binäre Mischungen (BM) mit 4-(trans-4-Pentylcyclohexyl)-benzonitril hergestellt. Die Schwellenspannung (V₁₀) und die Ansprechzeiten (tₒₙ und t_{off}) wurden in einer TN-Zelle (low bias tilt) mit 8 µm Plattenabstand bei 22°C gemessen; als Betriebsspannung wurde der 2,5-fache Wert der Schwellenspannung gewählt. Die entsprechenden Daten für 4-(trans-4-Pentylcyclohexyl)-benzonitril betragen: Klp. (N-I) 54,6°C, V₁₀ = 1,62V, tₒₙ = 22 ms, t_{off} = 42 ms, Δn = 0,120.

### BM-1

- 90 Gew.%: 4-(trans-4-Pentylcyclohexyl)-benzonitril
- 10 Gew.%: trans-4-(4-Cyclopentyl-butyl)-trans-4'-(3,4-difluorphenyl)-bicyclohexyl

Klp. (N-I) 57,9°C, V₁₀ = 1,62V, tₒₙ = 30 ms, t_{off} = 49 ms, Δn = 0,118.

### BM-2

- 80 Gew.%: 4-(trans-4-Pentylcyclohexyl)-benzonitril
- 20 Gew.%: trans-4-(4-Cyclopentyl-butyl)-trans-4'-(3,4-difluorphenyl)-bicyclohexyl

Klp. (N-I) 62,3°C, V₁₀ = 1,64V, tₒₙ = 34 ms, t_{off} = 56 ms, Δn = 0,113.

### BM-3

- 90 Gew.%: 4-(trans-4-Pentylcyclohexyl)-benzonitril
- 10 Gew.%: trans-4-(2-Cyclopentyl-ethyl)-trans-4'-(3,4,5-trifluorphenyl)-bicyclohexyl

Klp. (N-I) 56,6°C, V₁₀ = 1,51V, tₒₙ = 31 ms, t_{off} = 47 ms, Δn = 0,117.

### BM-4

- 80 Gew.%: 4-(trans-4-Pentylcyclohexyl)-benzonitril
- 20 Gew.%: trans-4-(2-Cyclopentyl-ethyl)-trans-4'-(3,4,5-trifluorphenyl)-bicyclohexyl

Klp. (N-I) 58,7°C, V₁₀ = 1,40V, tₒₙ = 41 ms, t_{off} = 55 ms, Δn = 0,114.

### BM-5

- 90 Gew.%: 4-(trans-4-Pentylcyclohexyl)-benzonitril
- 10 Gew.%: 4-Cyclopentyl-benzoesäure-4-cyano-3-fluor-phenyl-ester

Klp. (N-I) 50,1°C, V₁₀ = 1,35V, tₒₙ = 33 ms, t_{off} = 52 ms, Δn = 0,125.

### BM-6

- 80 Gew.%: 4-(trans-4-Pentylcyclohexyl)-benzonitril
- 20 Gew.%: 4-Cyclopentyl-benzoesäure-4-cyano-3-fluor-phenyl-ester

Klp. (N-I) 46,2°C, V₁₀ = 1,14V, tₒₙ = 45 ms, t_{off} = 67 ms, Δn = 0,126.

### BM-7

- 90 Gew.%: 4-(trans-4-Pentylcyclohexyl)-benzonitril
- 10 Gew.%: 4-(Cyclopentyl-methyl)-benzoesäure-4-cyano-3-fluorphenyl-ester

Klp. (N-I) 46,3°C, V₁₀ = 1,31V, tₒₙ = 34 ms, t_{off} = 56 ms, Δn = 0,120.

### BM-8

- 80 Gew.%: 4-(trans-4-Pentylcyclohexyl)-benzonitril
- 20 Gew.%: 4-(Cyclopentyl-methyl)-benzoesäure-4-cyano-3-fluorphenyl-ester

Klp. (N-I) 37,9°C, V₁₀ = 1,06V, tₒₙ = 50,2 ms, t_{off} = 82 ms, Δn = 0,114.

### BM-9

- 90 Gew.%: 4-(trans-4-Pentylcyclohexyl)-benzonitril
- 10 Gew.%: 4-(2-Cyclopentyl-ethyl)-benzoesäure-4-cyano-3-fluorphenyl-ester

Klp. (N-I) 49,9°C, V₁₀ = 1,42V, tₒₙ = 31 ms, t_{off} = 52 ms, Δn = 0,123.

### BM-10

- 80 Gew.%: 4-(trans-4-Pentylcyclohexyl)-benzonitril
- 20 Gew.%: 4-(2-Cyclopentyl-ethyl)-benzoesäure-4-cyano-3-fluorphenyl-ester

Klp. (N-I) 45,6°C, V₁₀ = 1,23V, tₒₙ = 42 ms, t_{off} = 68 ms, Δn = 0,124.

### BM-11

- 90 Gew.%: 4-(trans-4-Pentylcyclohexyl)-benzonitril
- 10 Gew.%: 4-(3-Cyclopentyl-propyl)-benzoesäure-4-cyano-3-fluorphenyl-ester

Klp. (N-I) 47,8°C, V₁₀ = 1,31V, tₒₙ = 36 ms, t_{off} = 57 ms, Δn = 0,120.

### BM-12

- 80 Gew.%: 4-(trans-4-Pentylcyclohexyl)-benzonitril
- 20 Gew.%: 4-(3-Cyclopentyl-propyl)-benzoesäure-4-cyano-3-fluorphenyl-ester

Klp. (N-I) 41,0°C, V₁₀ = 1,11V, tₒₙ = 50 ms, t_{off} = 81 ms, Δn = 0,116.

### BM-13

- 90 Gew.%: 4-(trans-4-Pentylcyclohexyl)-benzonitril
- 10 Gew.%: 4-(4-Cyclopentyl-butyl)-benzoesäure-4-cyano-3-fluorphenyl-ester

Klp. (N-I) 50,4°C, V₁₀ = 1,41V, tₒₙ = 32 ms, t_{off} = 54 ms, Δn = 0,123.

### BM-14

- 80 Gew.%: 4-(trans-4-Pentylcyclohexyl)-benzonitril
- 20 Gew.%: 4-(4-Cyclopentyl-butyl)-benzoesäure-4-cyano-3-fluorphenyl-ester

Klp. (N-I) 46,6°C, V₁₀ = 1,25V, tₒₙ = 45 ms, t_{off} = 74 ms, Δn = 0,123.

### BM-15

- 90 Gew.%: 4-(trans-4-Pentylcyclohexyl)-benzonitril
- 10 Gew.%: 4-(Cyclopentyl-methoxy)-benzoesäure-4-cyano-3-fluorphenyl-ester

Klp. (N-I) 51,4°C, V₁₀ = 1,39V, tₒₙ = 36 ms, t_{off} = 58 ms, Δn = 0,125.

### BM-16

- 80 Gew.%: 4-(trans-4-Pentylcyclohexyl)-benzonitril
- 20 Gew.%: 4-(Cyclopentyl-methoxy)-benzoesäure-4-cyano-3-fluorphenyl-ester

Klp. (N-I) 47,9°C, V₁₀ = 1,20V, tₒₙ = 51 ms, t_{off} = 84 ms, Δn = 0,126.

## Patentansprüche

1. Verbindungen der allgemeinen Formel 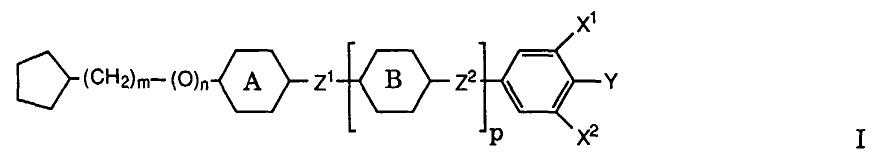 worin die
Ringe A und B unabhängig voneinander trans-1,4-Cyclohexylen oder gegebenenfalls einfach oder mehrfach mit Fluor substituiertes 1,4-Phenylen oder einer der Ringe A und B auch trans-1,3-Dioxan-5,2-diyl, Pyridin-5,2-diyl oder Pyrimidin-5,2-diyl;
Z¹ und Z² eine Einfachbindung oder eine der Gruppen auch -(CH₂)₂-, -CH₂O-, -COO- oder -C≡C-;
X¹ und X² Wasserstoff oder Fluor;
Y Fluor, Chlor, Cyano, -OCHF₂, -CHF₂, -OCF₃, oder -CF₃;
m eine ganze Zahl von 0 bis 7;
n 0 oder, falls A ein aromatischer Ring ist, auch 1; und
p 0 oder 1 bedeuten.

2. Verbindungen gemäss Anspruch 1, dadurch gekennzeichnet, dass Ring A trans-1,4-Cyclohexylen, gegebenenfalls einfach oder mehrfach mit Fluor substituiertes 1,4-Phenylen, trans-1,3-Dioxan-5,2-diyl, Pyridin-5,2-diyl oder Pyrimidin-5,2-diyl; und Ring B trans-1,4-Cyclohexylen oder gegebenenfalls einfach oder mehrfach mit Fluor substituiertes 1,4-Phenylen bedeutet.

3. Verbindungen gemäss Anspruch 1 oder 2, dadurch gekennzeichnet, dass p = 1 ist.

4. Verbindungen gemäss einem der Ansprüche 1 bis 3, der allgemeinen Formeln 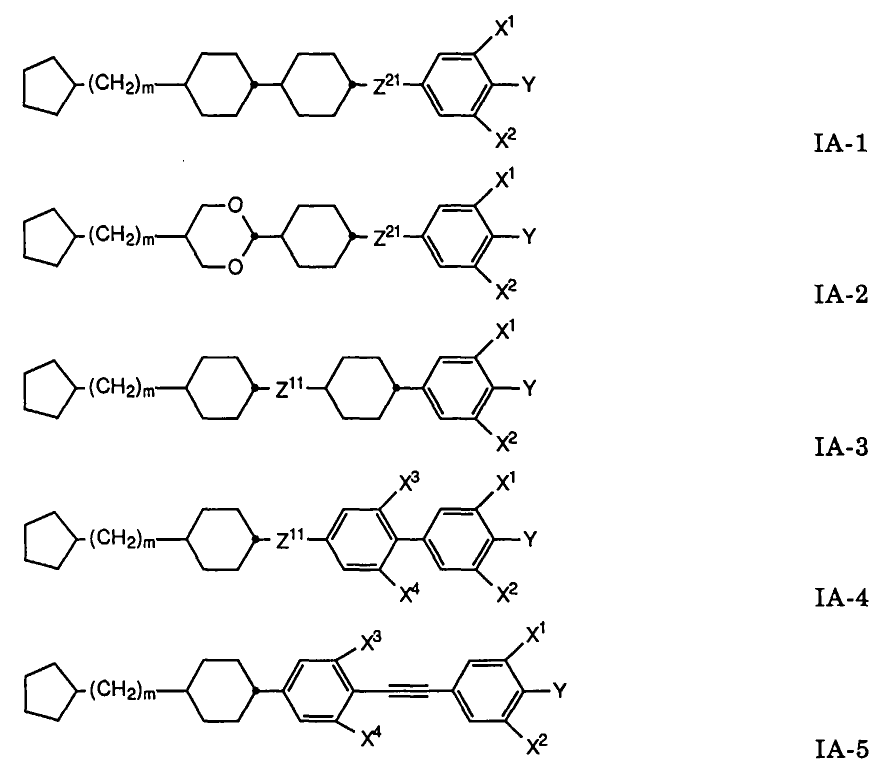 worin
Z¹¹ und Z²¹ eine Einfachbindung oder -(CH₂)₂-;
X³ und X⁴ unabhängig voneinander Wasserstoff oder Fluor bedeuten; und
m, Y, X¹ und X² die in Anspruch 1 angegebenen Bedeutungen haben.

5. Verbindungen gemäss einem der Ansprüche 1 bis 4, dadurch gekennzeichnet, dass m eine ganze Zahl von 0 bis 4 ist.

6. Verbindungen gemäss Anspruch 1 oder 2, dadurch gekennzeichnet, dass p = 0 ist.

7. Verbindungen gemäss einem der Ansprüche 1, 2 oder 6 der allgemeinen Formel 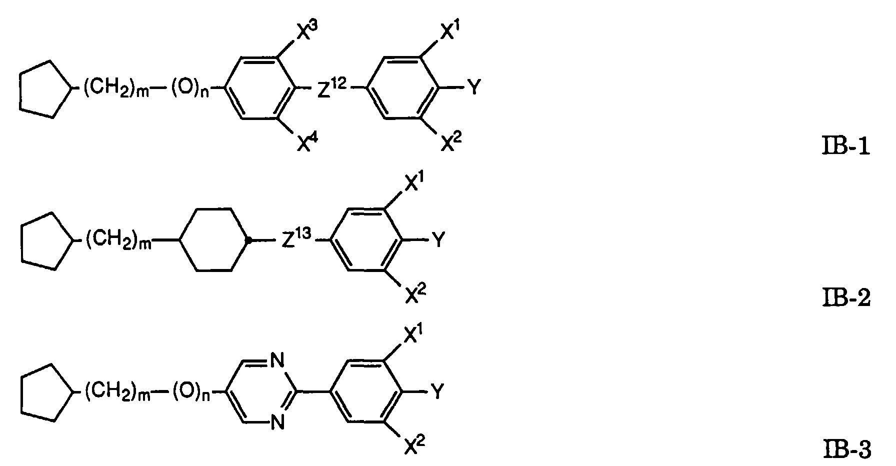 worin
m eine ganze Zahl von 0 bis 7 ist;
n 0 oder 1 ist;
X³ und X⁴ unabhängig voneinander Wasserstoff oder Fluor;
Z¹² eine Einfachbindung, -COO- oder -C≡C- und
Z¹³ eine Einfachbindung -(CH₂)₂-, -CH₂O- oder -COO- bedeuten; und
Y, X¹ und X² die in Anspruch 1 angegebenen Bedeutungen haben.

8. Verbindungen der Formel 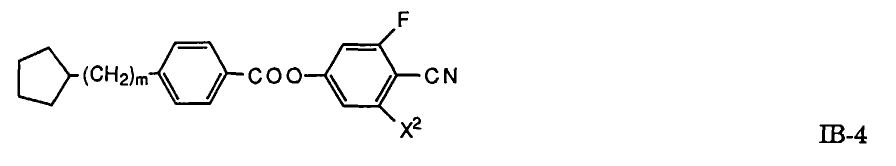 worin m und X² die in Anspruch 1 angegebenen Bedeutungen haben.

9. Flüssigkristallines Gemisch mit mindestens 2 Komponenten, dadurch gekennzeichnet, dass mindestens eine Komponente eine Verbindung der in Anspruch 1 definierten Formel I ist.

10. Flüssigkristallines Gemisch nach Anspruch 9, dadurch gekennzeichnet, dass der Anteil an Verbindungen der Formel I 1-40 Gew.-% beträgt.

11. Verwendung der Verbindungen der in Anspruch 1 definierten Formel I für elektro-optische Vorrichtungen.

12. Verwendung von flüssigkristallinen Mischungen gemäss einem der Ansprüche 9 oder 10 für elektro-optische Vorrichtungen.

13. Elektro-optische Anzeigevorrichtung enthaltend ein flüssigkristallines Gemisch gemäss Anspruch 9.
